# EUROPÄISCHE PATENTANMELDUNG

(11) **EP 2 196 139 A1**
(43) Veröffentlichungstag der Anmeldung: **16.06.2010**
(21) Anmeldenummer: 09015115.0
(22) Anmeldetag: 07.12.2009
(51) Int. Cl.: A61B 5/00

(54) **Intraorales Scangerät**

(30) Priorität: 09.12.2008 DE 102008060861
(71) Anmelder: Hintersehr, Josef, 64347 Griesheim (DE)
(72) Erfinder: Hintersehr, Josef, 64347 Griesheim (DE)
(74) Vertreter: Bill, Burkart Hartmut

(57) **Zusammenfassung**

Die Erfindung betrifft ein intraorales Scangerät zur Erfassung von dreidimensionalen Mess- bzw. Scandaten des Kiefers oder von Zähnen, welches in einem vorderen, in den Oralbereich einzuführenden Bereich, eine Scaneinheit beherbergt und welches an diesem vorderen Bereich zusätzlich eine Luftzufuhreinrichtung beherbergt, über welche unter Druck gesetzte Luft im Oralbereich lokal zuführbar ist.

## Beschreibung

Die Erfindung betrifft ein intraorales Scangerät.

Bekannt sind interorale Scangeräte, z. B. in Form eines in den Oralbereich einführbaren stiftartigen Scangeräts.

Üblicherweise ist in einem vorderen Bereich des Scangeräts eine Scaneinheit beherbergt, z. B. einer Laser-Scaneinheit, mittels welcher im Oralbereich dreidimensionale Mess- bzw. Scandaten des Kiefers oder von Zähnen etc. erfasst werden, wobei das Scangerät mit einer außerhalb des Oralbereichs angeordneten Datenverarbeitungsanlage in Verbindung steht, an welche die erfassten Mess- bzw. Scandaten zur weiteren, typischerweise CAD (Computer Aided Design) basierten, Datenverarbeitung übertragen werden.

Einsatz finden solche Scangeräte somit insbesondere bei Anwendung, bei denen solche dreidimensionale Kiefer- und/oder Zahndaten computergestützt zum Aufbau eines CAD-Modells einer gewünschten Dentalkomponente, wie einem Implantat und -aufbau einer Zahnkrone oder Zahnbrücke und Zahnbrückengerüst weiterverarbeitet werden und/oder zur Erzeugung von CAD-Modellschichten zu innerhalb eines Fräsens, Lasersinterverfahrens oder Laserschmelzverfahrens verwendbaren Schichtdaten weiterverarbeitet werden.

Als schwierig hat sich bei solchen gattungsbildenden intraoralen Scangeräten die Erfassung von qualifizierten dreidimensionalen Mess- bzw. Scandaten des Kiefers und/oder von Zähnen in Übergangsbereichen zum Backenfleisch oder Zahnfleisch (Gingiva) hin gezeigt, z.B. da dieser Bereich den Blick auf eine Sub Gingiva liegende Präparation verdeckt,

Aufgabe der Erfindung ist es daher ein solches gattungsbildendes intraoralen Scangeräts zur Erfassung von dreidimensionalen Mess- bzw. Scandaten innerhalb des Oralbereichs in noch effektiverer Weise weiterzubilden, insbesondere um die Qualität der Erfassung dreidimensionaler Mess- bzw. Scandaten des Kiefers und/oder von Zähnen in Übergangsbereichen zum Backenfleisch oder Zahnfleisch (Gingiva) zu verbessern durch Verdrängung de Zahnfleisches und so Offenlegungen der Sub Gingiva liegenden Präparation, Weiterer Vorteil ist, eventuell vorhandenen Speichel oder ausgetretenes Blut zu entfernen oder dessen Störung zu minimieren.

Die Erfindung löst die Aufgabe in überraschender Weise bereits durch einen Gegenstand mit den Merkmalen des anhängenden Anspruchs 1. Bevorzugte und/oder zweckmäßige Ausgestaltungen sind Gegenstand der Unteransprüche.

Demgemäß ist ein intraorales Scangerät zur Erfassung von dreidimensionalen Mess- bzw. Scandaten des Kiefers oder von Zähnen vorgeschlagen, welches in einem vorderen, in den Oralbereich einzuführenden Bereich, eine Scaneinheit beherbergt und welches an diesem vorderen Bereich zusätzlich eine Luftzufuhreinrichtung beherbergt, über welche unter Druck gesetzte Luft im Oralbereich lokal zuführbar ist. Hierdurch kann somit insbesondere in Übergangsbereichen zwischen harten, unnachgiebigen Zonen und weichen Zonen aufgrund des zugeführten Luftdrucks eine Tasche oder eine Art Nut ausgebildet werden, so dass auch in diesen Übergangsbereichen eine qualifizierte Erfassung von dreidimensionalen Mess- bzw. Scandaten harter, unnachgiebiger Zonen, wie insbesondere des Kiefers und/oder von zähnen ermöglicht ist da dies für die Kamera so deutlich sichtbar wird.

Die Erfindung wird nachfolgend anhand einer beispielhaften Ausführungsform unter Bezugnahme auf die Zeichnungen näher beschrieben. In den Zeichnungen zeigen:
Fig. 1 stark vereinfacht eine Ausführungsform der Erfindung beim Einsatz, und
Fig. 2 stark vereinfacht eine vergrößerte Ansicht eines Teilbereichs der Fig. 1.

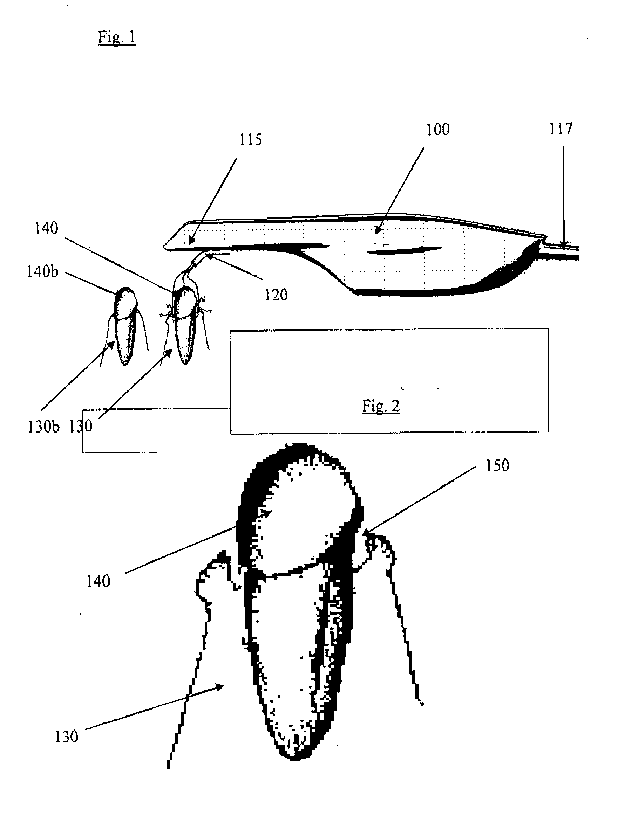

Die Figur 1 zeigt ein intraorales Scangerät 100, mit einem vorderen Bereich 115, der in einen nicht näher dargestellten Oralenbereich eingeführt ist, um dort dreidimensionale Mess- bzw. Scandaten zu erfassen, im dargestellten Fall von Zähnen 140, 140b.

Hierzu ist im vorderen, in den Oralbereich einzuführenden Bereich 115 des Scangeräts 100 eine nicht näher dargestellte Scaneinheit, z. B. eine Laser-Scaneinheit, beherbergt. Mit dieser können auf an und für sich einem Fachmann bekannte Weise dreidimensionale Mess- bzw. Scandaten des Kiefers oder von Zähnen etc. durch geeignete Handhabung des Scangeräts 100 erfasst werden.

Wie aus der Figur 1 ersichtlich, ist ferner am vorderen Bereich 115 eine Luftzufuhreinrichtung 120 beherbergt, über welche unter Druck gesetzte Luft im Oralbereich lokal zuführbar ist. Die Luft wird über den Verbindungsstrang 117 von außerhalb dem Scangerät 100 zugeführt, welcher folglich zumindest eine Art Röhre integriert.

Die Luftzufuhreinrichtung 120 bewirkt einen Luftstrom, welcher zweckmäßigerweise derart steuerbar ist, dass sich dieser voll umfänglich um den Bereich einer bestimmten harten, unnachgiebigen Zone ausbreiten kann und somit auch in Übergangsbereichen zwischen harten, unnachgiebigen Zonen und weichen Zonen aufgrund des zugeführten Luftdrucks eine Tasche oder eine Art Nut ausbildet, so dass auch in solchen Übergangsbereichen eine qualifizierte Erfassung von dreidimensionalen Mess- bzw. Scandaten harter, unnachgiebiger Zonen, wie insbesondere des Kiefers und/oder von Zähnen ermöglicht ist.

So ist Fig. 1 zu entnehmen, dass die Luftzufuhreinrichtung 120 zumindest einen Luftstrom bewirkt, der sich im Wesentlichen voll umfänglich um den Bereich eines sich außerhalb des Zahnfleisches 130 befindlichen Teils eines Zahns 140 ausbreitet. Bei Fig. 1 sind drei Düsen angedeutet, aus denen drei einzelne Luftströme entweichen, die sich in deren Gesamtheit im Wesentlichen voll umfänglich um diesen Bereich herum ausbreiten.

Hierdurch wird, wie insbesondere bei Figur 2 ersichtlich zwischen Zahnfleisch 130 und Zahn 140 eine Art Tasche oder Nut 150 ausgeformt, so dass auch der Übergangsbereich, der "normalerweise" durch das Zahnfleisch 130 vom Zahn 140 bedeckt ist von der Scaneinheit des Scangeräts 100 erfassbar ist. Mit anderen Worten wird also aufgrund des zugeführten Luftdrucks das Zahnfleisch 130 etwas vom Zahn 140 weg gedrückt, bildet folglich die Tasche oder Nut 150 aus und gibt somit an dieser Stelle den Zahn 140 frei.

Eine solche Tasche ist beispielsweise im Übergangsbereich zwischen Zahn 140b und Zahnfleisch 130b gemäß Fig. 1 nicht vorhanden.

Insbesondere um den Luftdruck gezielt zu steuern, z. B. um eine Tasche gleichzeitig rundherum um einen Zahn auszubilden oder größere und kleine Taschen auszubilden, ist es zweckmäßig, dass die Luftzufuhreinrichtung über mit einer Steuereinrichtung in Verbindung steht, mittels welcher die Menge und/oder der Druck der Luftzufuhr einstellbar ist.

Ferner ist es zum gezielten Ansteuern von bestimmten lokal auswählbaren Oralbereich zweckmäßig, dass die Luftzufuhreinrichtung wenigstens eine Düse besitzt, die zur gezielten Luftzufuhr in deren Lage verstellbar ist.

So kann z. B. eine Zone gezielt, d.h. lokal definiert und konzentriert, mit Luft angeströmt werden. Beispielsweise der Zahn 140 gemäß Fig. 1 wohingegen der Zahn 140b von der Luftströmung weitgehend ausgespart bleibt.

Eine solche Verstellung der wenigstens einen Düse kann hierbei je nach deren spezieller Ausbildung manuell, z. B. bevor die intraorale Erfassung durchgeführt wird, erfolgen oder mittels einer Steuereinrichtung, mit der die Luftzufuhreinrichtung in Verbindung steht. Im letzten Fall sind z. B. die Düsen über Aktoren am Scangerät verstellbar befestigt und in dem Verbindungsstrang 117 zusätzliche Leitungen zur Ansteuerung der Aktoren integriert.

Weiterer Vorteil ist, durch die Luftzufuhr eventuell vorhandenen Speichel oder ausgetretenes Blut zu entfernen oder dessen Störung zu minimieren.

Das Scangerät 100 steht darüber hinaus mit einer außerhalb des Oralbereichs angeordneten, nicht näher dargestellten Datenverarbeitungsanlage in Verbindung, im dargestellten Fall z. B. über in dem Verbindungsstrang 117 zusätzlich integrierte Leitungen, an welche die erfassten Mess- bzw. Scandaten zur weiteren, CAD (Computer Aided Design) basierten, Datenverarbeitung übertragen werden. Anstelle einer leitungsgebundenen Verbindung zwischen Scangerät 100 und der Datenverarbeitungsanlage kann jedoch auch eine schnurlose Verbindung vorgesehen werden. Auch muss eine solche Verbindung grundsätzlich nicht dauerhaft sein sondern im Scangerät kann z. B. ein Speicher untergebracht sein, aus welchem nach der intraoralen Erfassung die gespeicherten Daten ausgelesen werden.

## Patentansprüche

1. Intraorales Scangerät zur Erfassung von dreidimensionalen Mess- bzw. Scandaten des Kiefers oder von Zähnen, welches in einem vorderen, in den Oralbereich einzuführenden Bereich, eine Scaneinheit beherbergt und welches an diesem vorderen Bereich zusätzlich eine Luftzufuhreinrichtung beherbergt, über welche unter Druck gesetzte Luft im Oralbereich lokal zuführbar ist.

2. Intraorales Scangerät nach vorstehenden Anspruch, welches mit einer außerhalb des Oralbereichs angeordneten Datenverarbeitungsanlage in Verbindung steht, an welche die erfassten Mess- bzw. Scandaten zur weiteren, CAD (Computer Aided Design) basierten, Datenverarbeitung übertragen werden.

3. Intraorales Scangerät nach vorstehenden Anspruch, wobei die Verbindung leitungsgebunden oder schnurlos ist.

4. Intraorales Scangerät nach einem der vorstehenden Ansprüche, wobei die Luftzufuhreinrichtung mit einer Steuereinrichtung in Verbindung steht, mittels welcher die Menge und/oder der Druck der Luftzufuhr einstellbar ist.

5. Intraorales Scangerät nach einem der vorstehenden Ansprüche, wobei die Luftzufuhreinrichtung wenigstens eine Düse besitzt, die zur gezielten Luftzufuhr in deren Lage verstellbar ist.

6. Intraorales Scangerät nach vorstehendem Anspruch, wobei die wenigstens eine Düse manuell oder mittels einer Steuereinrichtung, mit der die Luftzufuhreinrichtung in Verbindung steht verstellbar ist.
